Europäisches Patentamt

European Patent Office

Office européen des brevets

(19)

(11) Numéro de publication: **0 126 666**
**B1**

# FASCICULE DE BREVET EUROPÉEN

(12)

(45) Date de publication du fascicule du brevet:
23.09.87

(51) Int. Cl.⁴: **A 23 L 1/305**, A 61 K 37/18

(21) Numéro de dépôt: **84400806.0**

(22) Date de dépôt: **20.04.84**

(54) Nouvelles compositions utilisables en diététique, réanimation et thérapeutique, renfermant une fraction protéique à base de 3 types de minipeptides et leurs applications.

(30) Priorité: 20.04.83 FR 8306444

(43) Date de publication de la demande:
28.11.84 Bulletin 84/48

(45) Mention de la délivrance du brevet:
23.09.87 Bulletin 87/39

(84) Etats contractants désignés:
AT BE CH DE FR GB IT LI LU NL SE

(56) Documents cité:
EP-A-0 034 083
EP-A-0 049 666
EP-A-0 055 172
EP-A-0 065 663
US-A-3 697 287
US-A-3 698 912

CHEMICAL ABSTRACTS, vol. 86, no. 5, 31 janvier 1977, page 148, no. 27231p, Columbus, Ohio, USA; D.W. WEST et al.: "A study of the enzymic dephosphorylation of beta-casein and a derived phosphopeptide"

(73) Titulaire: ROUSSEL- UCLAF, 35, boulevard des Invalides, F-75007 Paris (FR)

(72) Inventeur: Mendy, François, 24, quai du 4 Septembre, F-92100 Boulogne (FR)

(74) Mandataire: Fritel, Hubert, Département des Brevets ROUSSEL UCLAF B.P. no 9, F-93230 Romainville (FR)

EP 0 126 666 B1

## Description

La présente invention concerne de nouvelles compositions destinées à être utilisées en diététique, en réanimation et en thérapeutique, renfermant une fraction protéique à base de 3 types de minipeptides et leurs applications.

La présente invention a pour objet des compositions contenant des glucides, des vitamines, des sels minéraux, une fraction lipidique et une fraction protéique, caractérisées en ce que la fraction protéique renferme les 3 types de minipeptides suivants:

- minipeptides de lactosérum,
- minipeptides de caséine totaux,
- minipeptides de caséine sans phosphopeptide.

Les minipeptides de lactosérum peuvent être obtenus par hydrolyse enzymatique totale de protéines de lactosérum. Ce procédé d'hydrolyse enzymatique et les minipeptides obtenus par ledit procédé sont décrits dans le brevet européen publié sous le n° 0 022 019.

Les deux autres types de minipeptides contenus dans la fraction protéique des compositions de l'invention peuvent être préparés à partir de phosphocaséinates de cations monovalents ou bivalents, traités selon les procédés indiqués dans les 2 brevets européens publiés sous les numéros 0 033 686 et 0 034 083.

Ces deux brevets indiquent, en premier lieu, comment les minipeptides de caséine totaux sont obtenus par hydrolyse enzymatique des phosphocaséinates de cations monovalents ou bivalents. Puis, ces 2 brevets décrivent les différentes étapes permettant de séparer à partir des minipeptides de caséine totaux, les phosphopeptides d'une part, et les minipeptides sans phosphopeptides d'autre part.

De nombreux produits à base de minipeptides utilisés en diététique ou en thérapeutique et plus particulièrement, en nutrition thérapeutique existent déjà sur le marché. Ces produits possèdent une fraction protéique contenant, en proportions variables, des acides aminés libres et des minipeptides à chaînes plus ou moins longues. Il y a quelques années, n'existaient que des produits à base d'acides aminés, mais, de plus en plus, on préfère des produits à base de petits peptides. En effet, les produits à base d'acides aminés sont toujours hyper-osmolaires, et ainsi, conduisent souvent à des intolérances digestives. Des recherches cliniques, comme par exemple, celles de SILK D., PERRETT D., CLARK M., "Intestinal transport of two dipeptides containing the same two neutral amino acids in man", Clin. Sci. Mol. Med. 45: 291-299, 1973, ont montré, de plus, la meilleure absorption des petits peptides par rapport aux acides aminés libres.

Le premier avantage des compositions selon l'invention est donc de contenir une fraction protéique ne renfermant que des minipeptides et pratiquement dépourvue d'acides aminés libres (présents dans une proportion inférieure à 15 %).

En outre, les travaux de KEOHANE P., BROWN Barbara, GRIMBLE G., SILK D., "The peptide nitrogen source of elemental diets - comparisons of absortive properties of five partial enzymic hydrolysates of whole protein", A.S.P.E.N. 6th Clinical Congress, 3-6 février 1982, San Francisco, ont montré la meilleure absorption des petits peptides lorsque ceux-ci proviennent d'un mélange contenant, entre autres, des peptides de caséine et des peptides de lactosérum, par rapport à des peptides ayant une autre origine.

Les compositions selon l'invention présentent le second avantage d'associer des minipeptides, qui par leur nature, sont très bien absorbés par l'organisme.

Cependant un mélange protéique à base de minipeptides de caséine et de minipeptides de lactosérum dans une composition destinée à la diététique, à la réanimation ou à la thérapeutique amènerait:

- soit un taux d'acides aminés soufrés trop élevé si les minipeptides de lactosérum étaient en quantité trop importante;
- soit un taux de phosphore lié aux protéines trop élevé si à l'inverse, les minipeptides de caséine étaient en quantité trop importante.

Il fallait donc réaliser un mélange de minipeptides de caséine et de minipeptides de lactosérum d'une part, respectant l'équilibre nécessaire entre ces 2 types de minipeptides et d'autre part, assurant un taux de phosphore organique lié aux protéines et un taux d'acides aminés soufrés adéquats.

Or, seuls les minipeptides de caséine apportent du phosphore lié aux protéines, les minipeptides de lactosérum n'en contiennent pas. La nécessité d'un équilibre entre minipeptides de caséine et minipeptides de lactosérum conduit à un apport en phosphore lié aux protéines trop élevé.

Il était donc intéressant de réaliser des compositions selon l'invention dans lesquelles une partie des minipeptides de caséine totaux étaient remplacés par des minipeptides sans phosphopeptide afin de diminuer dans ces compositions le taux de phosphore provenant de la caséine lié aux protéines.

De cette façon, la teneur en phosphore lié aux protéines est donc réajustée et en même temps, la teneur en acides aminés soufrés est réglée au niveau souhaité.

Par ailleurs, de l'apport en acides aminés soufrés et de l'apport en phosphore lié aux protéines dépend la charge en ions $H^+$, paramètre important chez les malades auxquels sont destinés les compositions de l'invention (malades très souvent en insuffisance rénale fonctionnelle, aux possibilités de régulation de l'équilibre acido basique déficientes).

L'ajustement du taux de phosphore organique dans les compositions selon l'invention permet donc aussi, plus globalement, de moduler la charge en ions $H^+$ des compositions.

La présente invention réalise donc des compositions dans lesquelles les 3 types de minipeptides associées permettent de moduler le taux de phosphore lié aux protéines.

Le taux de phosphore lié aux protéines ainsi obtenu dans ces compositions est variable et dépend de la proportion de la partie phosphoprotéique contenue dans ces compositions. Pour obtenir un taux de phosphore organique physiologique, la fraction phosphoprotéique doit représenter de préférence de 1 % à 10 % en poids des compositions de l'invention.

Ces précédentes conditions permettent notamment de réaliser une association des 3 types de minipeptides conduisant à des compositions dans lesquelles le taux de phosphore lié aux protéines est au minimum égal à celui présent dans le lait de femme, c'est-à-dire de l'ordre de 1,5 mg par g de protéine et au maximum égal à celui présent dans le lait de vache, c'est-à-dire de l'ordre de 6,6 mg par g de protéine.

La présente invention a donc notamment pour objet des compositions caractérisées en ce que le poids de la fraction protéique représente 1 % à 10 % du poids total et caractérisées en ce que le taux de phosphore lié aux protéines est compris entre 1,5 mg par g de protéine et 6,6 mg par g de protéine.

Par ailleurs, suivant les quantités respectives des 3 types de minipeptides, la distribution en acides aminés des compositions de l'invention est variable. Ainsi, les 3 types de minipeptides associés permettent d'obtenir des aminogrammes différents adaptés à des situations pathologiques spécifiques.

L'association des 3 types de minipeptides, notamment en quantités sensiblement égales, dans la fraction protéique des compositions selon l'invention, permet de disposer d'une distribution en acides aminés assurant une efficacité nutritionnelle maximale.

La présente invention concerne donc notamment des compositions caractérisées en ce que les 3 types de minipeptides sont présents sensiblement en quantités pondérales équivalentes.

Dans ces conditions, le taux de phosphore lié aux protéines est environ égal à 3 mg/g de protéine.

L'invention concerne donc notamment des compositions caractérisées en ce que le taux de phosphore lié aux protéines est environ égal à 3 mg/g de protéine et tout particulièrement, des compositions caractérisées en ce que la distribution en acides aminés de la fraction protéique est celle donnée ci-après, les chiffres suivants étant exprimés en gramme d'acide aminé pour 100 g d'acides aminés:

| Ile | 5,1 | Val | 5,9 |
|-----|-----|-----|-----|
| Leu | 10,1 | Arg | 3,3 |
| Lys | 8,5 | His | 2,5 |
| Met | 2,3 | Ala | 3,6 |
| Cys | 1,6 | Asp | 8,4 |
| Phe | 4,6 | Glu | 15,9 |
| Tyr | 4,9 | Gly | 1,8 |
| Thr | 4,4 | Pro | 8,4 |
| Trp | 1,8 | Ser | 4,7 |

Cet aminogramme permet ainsi d'augmenter le rapport en acides aminés ramifiés tout en conservant un taux de lysine élevé.

Bien entendu, les compositions de l'invention apportent tous les éléments nutritifs nécessaires, autres que protéiques, en proportions étudiées. Elles renferment ainsi des glucides assurant 50 à 60 % de l'apport énergétique total (= A.E.T), des vitamines (A, D, E, C, $B_1$, $B_2$, PP, $B_6$, $B_{12}$, acide folique, biotine, $B_5$, $K_1$, choline).

Les compositions selon l'invention assurent aussi une couverture des besoins physiologiques en éléments minéraux.

Elles renferment également une fraction lipidique assurant 30 % à 35 % de l'A.E.T.

L'invention a notamment pour objet des compositions caractérisées en ce que leur fraction lipidique a approximativement la composition suivante:

| - triglycérides à chaîne moyenne | 56,4 % |
|-----|-----|
| - huile d'Oenothera | 25,6 % |
| - huile de soja | 18%. |

Plus de la moitié de cette fraction lipidique est composée de triglycérides à chaîne courte ou moyenne. Ceci permet une bonne tolérance même chez les malades souffrant d'une malabsorption ou d'une maldigestion des lipides ou d'une mauvaise métabolisation des acides gras essentiels. En effet, les triglycérides à chaîne courte et moyenne sont facilement assimilables par l'organisme même dans le cas où la digestion est perturbée.

Cette fraction lipidique assure l'apport en acides gras essentiels suivants:
- acide linoléique (= 9,6 % de l'A.E.T.),
- acide γ-linoléique (= 0,8 % de l'A.E.T.),
- acide α-linoléique (= 0,4 % de l'A.E.T.).

Les acides nommés ci-dessus ou leurs dérivés polyinsaturés supérieurs habituels peuvent être ajoutés spécifiquement.

Cette adjonction peut être réalisée par l'addition de triglycérides de synthèse de 2 sortes:
- triglycérides de synthèse en position 1, 2 ou 3 de l'acide linoléique ou γ-linoléique (C18:3,ω6), de l'acide dihomo α-linoléique (C20:3,ω6), de l'acide arachidonique (C20:4,ω6), de l'acide docosatétraénoïque (C22:4,ω6), ou de l'acide docosapentaénoïque (C22:5,ω6);
- triglycérides de synthèse en position 1, 2 ou 3 de l'acide α-linoléique (C18:3,ω3), de l'acide eicosatétraénoïque (C20:4,ω3), de l'acide eicosapentaénoïque (C20:5,ω3), de l'acide docosahexaénoïque (C22:6,ω3) ou de l'acide docosapentaénoïque (C22:5,ω3).

Les compositions de l'invention sont utilisables en diététique, réanimation et thérapeutique.

La demande a ainsi pour objet l'application des compositions telles que définies précédemment comme aliment ou supplément alimentaire répondant à des besoins spécifiques nutritionnels.

Les compositions de l'invention sont utilisables lorsqu'une assistance nutritionnelle post-opératoire est nécessaire. Elles sont aussi utilisables en nutrition préopératoire, en particulier lorsqu'un régime sans résidu est souhaité. En effet, les compositions de l'invention sont complètement absorbées dans la partie proximale de l'intestin grêle, ne laissant qu'un résidu fécal minime.

Les compositions de l'invention peuvent aussi être employées en cas de pathologie du tube digestif, et notamment, lorsque la surface intestinale est réduite ou présente des affections pathologiques. C'est le cas par exemple, des affections caractérisées par une inflammation chronique associée à des fistules, ou par un trouble de l'absorption dû à une diminution de la surface de l'intestin grêle. C'est aussi le cas de la maladie de Crohn, des maladies intestinales chroniques.

Les compositions de l'invention trouvent également leur emploi lorsqu'il existe des troubles de la sécrétion digestive entraînant une mauvais digestion ou malabsorption:
- pancréatite aigüe,
- syndrome de malabsorption dû à une réticulose maligne,
- certaines maladies de système,
- trouble ischémiques de l'intestin.

Par ailleurs, chaque fois qu'il y a une atteinte inflammatoire de l'intestin, un risque d'allergisation à des protéines entières existe. Les compositions de l'invention peuvent donc aussi être employées pour éviter la survenue d'allergies au cours de maladies inflammatoires de l'intestin.

La demande a ainsi pour objet les aliments ou suppléments alimentaires ainsi que les produits de nutrition thérapeutique contenant les compositions telles que définies précédemment en association éventuelle avec un véhicule neutre, convenant à l'administration orale ou entérale.

La présente demande a également pour objet des compositions, telles que définies précédemment pour utilisation comme médicament. Les nouvelles compositions de l'invention constituent, en raison de leurs propriétés, des médicaments très utiles dans le traitement des affections décrites plus haut.

La dose usuelle des compositions de l'invention varie en fonction de l'état du malade, de l'affection en cause et de la voie d'administration choisie.

On peut, par exemple, administrer une dose de composition de l'invention assurant un apport protéique d'environ 47 g à 118 g et de préférence, d'environ 71 g, un apport lipidique d'environ 58,5 g à 146 g et de préférence, d'environ 88 g, un apport glucidique d'environ 197 g à 493 g et de préférence, d environ 296 g, ces doses étant comprises par jour et par voie orale chez un adulte.

La présente demande a enfin pour objet les préparations pharmaceutiques contenant un médicament ci-dessus, en association éventuelle avec un véhicule neutre usuel convenant à l'administration orale ou entérale.

Ces préparations pharmaceutiques reuvent se présenter sous les formes couramment utilisés en médecine humaine, soit sous forme liquide, soit sous forme de poudre et sont contenues par exemple, dans des boîtes métalliques, des flacons ou des poches ou des bouteilles plastiques.

Dans les préparations données ci-après en exemples, 1 à 5 g des maltodextrines peuvent être remplacés par du galactose.

Les exemples donnés ci-après illustrent l'invention.

## EXEMPLE 1

On a préparé la préparation diététique liquide à usage oral ou entéral, de formule suivante:

**Composition centésimale**

| - glucides: | 13,15 | g | (maltodextrines) | | |
| - lipides: | 3,9 | g | | | |
| | dont | - | triglycérides à chaîne moyenne | 2,2 | g |
| | | - | huile d'Oenothera | 1,0 | g |
| | | - | huile de soja | 0,7 | g |
| - protéines: | 3,15 | g | | | |
| | dont | - | minipeptides de lactosérum | 1,05 | g |
| | | - | minipeptides de caséine totaux | 1,05 | g |
| | | - | minipeptides de caséine sans phosphopeptide | 1,05 | g |

0 126 666

- vitamines: selon les normes américaines des RDA et des ESADDI
  A, D, E, C, $B_1$, $B_2$, PP, $B_6$, $B_{12}$, acide folique, biotine, $B_5$, $K_1$, choline;
  - mineraux: selon les normes américaines des RDA et des ESADDI
  calcium, phosphore, magnésium, fer, zinc, iode, sodium, potassium, chlorure, cuivre, manganèse, sélénium.......0,404 g
  - véhicule QSP.........................100 ml.
  - R.D.A.: "Recommended Dietary Allowances"
  9ème Edition 1980
  Committee on Dietary Allowances,
  Food and Nutrition Board
  Division of Biological sciences, National Research Council, National Academy of Sciences, Washington, D.C., 1980
  - E.S.A.D.D.I.: "Estimated Safe and Adequate Daily Dietary Intakes"
  (Nutrition Reviews, Vol 38, N° 8 August 1980, p 291) (Journal of The American Dietetic Association, Vol 76, N° 3, mars 19).
  A titre indicatif, la fraction lipidique de cette préparation assure l'apport en acides gras essentiels suivant:
  - acide linoléïque: 1,1 g,
  - acide $\gamma$-linoléïque: 0,08 g,
  - acide $\alpha$-linoléïque: 0,04 g.

## EXEMPLE 2

On a préparé la préparation diététique liquide à usage oral ou entéral, de formule suivante:
**Composition centésimale**

| - glucides: | 15,6 | g | (Maltodextrines) | | |
|---|---|---|---|---|---|
| - lipides: | 5,2 | g | | | |
| | dont | - | triglycérides à chaîne moyenne | 2,9 | g |
| | | - | huile d''Oenothera | 1,2 | g |
| | | - | huile de soja | 1,05 | g |
| | | - | lécithine | 0,05 | g |
| - protéines: | 6 | g | | | |
| | dont | - | minipeptides de lactosérum | 2 | g |
| | | - | minipeptides de caséine totaux | 2 | g |
| | | - | minipeptides de caséine sans phosphopeptides | 2 | g |

- vitamines: selon les normes amércaines des R.D.A. et des E.S.A.D.D.I. A, E, C, $B_1$, $B_2$, PP, $B_6$, $B_{12}$, acide folique, Biotine, $B_5$, choline
- minéraux: selon les normes américaines des R.D.A. et des E.S.A.D.D.I.
calcium, phosphore, magnésium, sodium, potassium, ion chlorure, fer, zinc, cuivre, manganèse, iode, sélénium......... 0,562g
- véhicule.................................. q.s.p. 100 ml.
La préparation de 7500 litres de composition de l'exemple 1 (7500 litres répartis ensuite dans das boîtes métalliques) est réalisée en 3 étapes comme suit:
**1ère étape: préparation d'une émulsion lipidique**
Le matériel utilisé est constitué de:
- 2 cuves en acier inoxydables de 2000 litres munies:
. d'un système d'agitation,
. d'un système de chauffage et refroidissement,
. d'un dispositif permettant de faire le vide,
. d'une pompe centrifuge,
. d'une pompe haute pression.
Dans une cuve de 2000 litres, on introduit 1500 litres d'eau osmosée et on commence à chauffer sous agitation.
Lorsque la température atteint 65°C, on incorpore:
- 75 kg d'huile d'Onagre,
- 37 kg 50 de lécithine de soja,
- 97 kg 500 de T.C.M.,
- 37 kg 500 de monostéarate de glycérol.
On laisse sous agitation, débute le refroidissement et ajoute les vitamines E, A, D prédissoutes dans 48 kg 750 d'huile de soja.
Lorsque la température est de l'ordre de 20°C, on débute le premier passage sur la pompe haute pression.

5

La pression est ajustée à 50 kg/cm$^2$ au deuxième étage de la pompe et à 250 kg/cm$^2$ au premier.

A la sortie, le produit est récupéré dans la seconde cuve.

On procède de cette façon à 4 passages sur la pompe haute pression en passant alternativement d'une cuve à l'autre.

Un contrôle rapide de la taille des Liposomes de l'émulsion peut être effectué au Coulter Counter.

**2ème étape: préparation d'une phase amidonnée**

Le matériel utilisé est constitué:

- d'une cuve en acier inoxydable de 8000 litres munie:
. d'un dispositif d'agitation,
. d'un système permettant de faire le vide,
- d'une cuve de 500 litres en acier inoxydable munie:
. d'une turbine de mélange,
- d'un échangeur à plaque doté de 3 circuits:
. un chauffage,
. un refroidissement à 15°C,
. un refroidissement à 5°C,
- de 2 pompes centrifuges de 20 m$^3$ / heure.

Le mélangeur étant en circuit fermé avec la cuve, les pompes et l'échangeur, on incorpore 3700 litres d'eau osmosée et on la chauffe à 85°C.

Lorsque cette température est atteinte, on incorpore par le mélangeur 195 kg de fécule déshydratée (1 à 4 % selon la viscosité souhaitée) et on maintient la température à 85°C pendant 30 mn.

A l'issue de cette latence, on abaisse la température à 50°C grâce à la section refroidissement de l'échangeur puis on incorpore successivement:

- 98 kg de minipeptides de la lactosérum,
- 92 kg de minipeptides de caséine totaux,
- 100 kg 500 de minipeptides de caséine sans phosphopeptides,
- 850 kg de malto dextrines.

On baisse la température à 40°C et introduit:

- le chlorure de magnésium,
- l'hydrogénophosphate de potassium,
- le chlorure de calcium,
- le chlorure de sodium,
- le chlorure de choline,
- l'iodure de potassium et le sélénite de sodium.

On refroidit la température du mélange à 25°C et on ajoute:

- l'acide ascorbique,
- le sulfate de fer,
- le nicotinamide ou vitamine <u>PP</u>,
- le citrate de zinc
- le pantothénate de calcium,
- le sulfate de manganèse,
- le chlorhydrate de pyridoxine ou vitamine B$_6$,
- le chlorhydrate de thiamine ou vitamine B$_1$,
- le sulfate de cuivre,
- la vitamine B$_{12}$ 1 % sur mannitol,
- la riboflavine ou vitamine B$_2$,
- l'acide folique,
- la biotine.

**3ème étape: préparation du mélange final**

La phase amidonnée étant sous agitation dans la cuve de 8000 litres, on introduit l'émulsion lipidique préparée à la 1ère étape grâce à une pompe centrifuge, ajuste le niveau de la cuve à 7450 litres avec de l'eau osmosée et le pH à 7,10 grâce à une solution d'hydroxyde de potassium, vérifie ensuite le niveau de la cuve et effectue une mesure de la teneur en matière sèche dans la cuve, fait le vide final et incorpore de l'azote dans la cuve.

Le mélange final ainsi obtenu est ensuite stérilisé par filtration puis à 150°C pendant 8 secondes par un passage dans un circuit de serpentins.

Le produit final est ensuite réparti respectivement dans des boîtes métalliques de 375 ml; celles-ci étant bouchées aseptiquement (les boîtes et couvercles sont préalablement stérilisés grâce à la vapeur surchauffée).

**Etude clinique du produit de l'exemple 1**

6 malades, âgés de 15 à 53 ans ont été suivis pendant une période de 14 jours en milieu de réanimation gastroentérologique. Ces malades présentaient une pathologie gastrointestinale grave avec une ou plusieurs complications (par exemple: chlolécystite aigüe avec pancréatite oedémateuse; plaie par balle ayant entrainée une plaie duodénale, une plaie du foie, une plaie du côlon droit; pancréatite aigüe avec syndrome toxi-infectieux).

6

Le but de cette étude était triple:

1) Etudier la tolérance du produit;
2) Evaluer l'absorption,
3) Apprécier l'impact nutritionnel du produit.

Pendant toute la durée de cette étude, les 6 malades ont reçu une alimentation entérale exclusive par sonde à débit continu, 24 heures sur 24.

Cette alimentation était composée exclusivement du produit de l'exemple 1 pour tous les malades, sauf pour un des malades pour lequel une adjonction d'eau de riz a été faite en raison d'un syndrome toxi-infectieux sévère.

Le tableau suivant indique pour chaque malade:
- l'apport énergétique par 24 heures en Kcal,
- le volume de produit de l'exemple 1 administré par 24 heures,
- l'apport quotidien en azote (g/24 h.) correspondant à l'administration du support nutritionnel.

| Malade | 1 | 2 | 3 | 4 | 5 | 6 |
|---|---|---|---|---|---|---|
| Apport énergétique Kcal/24 h. | 4125 | 4125 | 4125 | 2300 | 3750 | 3750 |
| Volume de produit de l'exemple 1 en ml par 24 h. | 4125 | 4125 (11 boites de 375ml) | 4125 | 2250 (6 b. de 375 ml) | 3750 (10 boîtes de 375ml) | 3750 |
| Apport en azote g/24 h. | 22 | 22 | 22 | 12 | 20 | 20 |

### 1) Etude de la tolérance du produit

Ont été retenus comme critères de tolérance:
- acceptabilité,
- vomissements,
- douleurs abdominales,
- diarrhées
- ballonnements.

Selon ces critères, sous un apport de 3750 à 4125 ml de produit par jour, les malades 1, 2, 3, 5 et 6 ont présenté une excellente tolérance.

Pour le malade 4, les critères de tolérance définis ci-dessus étaient également bons, avec une diminution de l'apport à 2300 ml/jour du produit.

### 2) Etude de l'absorption et de l'efficacité nutritionnelle

La valeur nutritionnelle du produit de l'exemple 1 a été étudiée à l'aide des résultats suivants (voir tableau I):

- Le coefficient d'absorption des minipeptides du produit de l'exemple 1, même dans le cas des pathologies gastro-entérologiques sévères et d'une toxi-infection, a été supérieur à 95 % dans tous les cas sauf un.

De plus, dans ce groupe de malades atteints de troubles gastro-entérologiques sévères qui, dans certains services, font éviter toute nutrition entérale, le coefficient d'absorption est nettement supérieur à la moyenne des coefficients d'absorption des malades habituels du service.

- Le bilan azoté se révèle positif dès le deuxième jour, variant entré + 9,25 et + 15 g/24 h., sauf pour le malade 4 (+ 3 g). Ce malade présentait une toxi-infection nécessitant une réduction d'apport (12 g d'azote/24 h.).

C'est ainsi que, même avec un apport calorico azoté nettement diminué, l'efficacité biologique du produit a permis de maintenir un bilan azoté positif.

- Albumine: un des critères principaux du métabolisme protidique est le taux d'albumine plasmatique. Ce taux d'albumine a augmenté de façon significative au cours du traitement.

De même, la prise de poids a augmenté de façon significative (2,5 kg).

Dans cette étude, tous les autres paramètres métaboliques sont restés identiques ou ont augmenté dans les limites de la normale ou bien se sont améliorés.

Enfin, l'évolution clinique est excellente pour les 6 malades.

Les critères nutritionnels et l'état clinique se sont donc améliorés de façon parallèle au bilan azoté.

**TABLEAU I**

| MALADE | AGE | COEFFICIENT D'ABSORPTION DES MINIPEPTIDES (J0) | | ALBUMINE J14 | POIDS J0 | J14 | AUTRES PARAMETRES METABOLIQUES | RESULTATS CLINIQUES |
|--------|-----|------|------|------|------|------|------|------|
| 1. | 52 | 96,5 % | 31 | 33,4 | 60 | 60 | Tous les paramètres sont restés ou montés dans les limites du normal ou se sont améliorés. Le bilan azoté s'est positivé dès le 2ème jour. | Excellente progression avec guérison. |
| 2. | 51 | 98,1 % | 30,8 | 39 | 71 | 75 | idem | idem |
| 3. | 47 | 98,7 % | 36,5 | 43,7 | 70 | 72 | idem | idem |
| 4. | 44 | 95,5 % | 38 | 45,9 | 64 | 58 | idem | Importante toxi-infection contrôlée et guérison. |
| 5. | 15 | 86,5 % | 33 | 43,6 | 43 | 45 | idem | Excellente progression avec guérison. |
| 6. | 16 | 97,5 % | 41 | 48 | 68 | 70 | idem | idem |

**Conclusion**

Le produit de l'exemple 1, utilisé dans cet essai clinique sur des malades présentant une pathologie gastrointestinale sévère avec une ou plusieurs complications, a présenté une efficacité nutritionnelle excellente qui a permis d'assurer.

- une stabilisation ou une amélioration des paramètres biologiques dans les limites de la normale;
- un coefficient d'absorption des minipeptides supérieur à 95 %;
- un bilan azoté nettement positif;
- une amélioration de l'état clinique.

Par ailleurs, ce produit présente une très bonne tolérance.

**Revendications**

1) Compositions contenant des glucides, des vitamines, des sels minéraux, une fraction lipidique et une fraction protéique, caractérisées en ce que la fraction protéique renferme les 3 types de minipeptides suivants:
- minipeptides de lactosérum,
- minipeptides de caséine totaux,
- minipeptides de caséine sans phosphopeptides.

2) Compositions selon la revendication 1, caractérisées en ce que le poids de la fraction protéique représente 1 % à 10 % du poids total.

3) Compositions selon la revendication 1 ou 2, caractérisées en ce que le taux de phosphore lié aux protéines est compris entre 1,5 mg par g de protéine et 6,6 mg par g de protéine.

4) Compositions selon l'une quelconque des revendications 1 à 3, caractérisées en ce que les 3 types de minipeptides sont présents sensiblement en quantités pondérales équivalentes.

5) Compositions selon la revendication 4, caractérisées en ce que le taux de phosphore lié aux protéines est environ égal à 3 mg/g de protéine.

6) Compositions selon la revendication 4, caractérisées en ce que la distribution en acides aminés de la fraction protéique est celle donnée ci-après, les chiffres suivants étant exprimés en gramme d'acide aminé pour 100 g d'acides aminés:

| Ile | 5,1 | Val | 5,9 |
|-----|-----|-----|-----|
| Leu | 10,1 | Arg | 3,3 |
| Lys | 8,5 | His | 2,5 |
| Met | 2,3 | Ala | 3,6 |
| Cys | 1,6 | Asp | 8,4 |
| Phe | 4,6 | Glu | 15,9 |
| Tyr | 4,9 | Gly | 1,8 |
| Thr | 4,4 | Pro | 8,4 |
| Trp | 1,8 | Ser | 4,7 |

7) Compositions selon l'une quelconque des revendications 1 à 6, caractérisées en ce que leur fraction lipidique a approximativement la composition suivante:
- triglycérides à chaîne moyenne 56,4 %
- huile d'Oenothera 25,6 %

- huile de soja 18 %

8) Utilisation des compositions selon l'une quelconque des revendications 1 à 7, comme aliment ou supplément alimentaire répondant à des besoins spécifiques nutritionnels.

9) Aliment ou supplément alimentaire contenant des compositions selon l'une quelconque des revendications 1 à 7 en association éventuelle avec un véhicule neutre convenant à l'administration orale ou entérale.

10) Compositions selon l'une quelconque des revendications 1 à 7, pour utilisation comme produits de nutrition thérapeutique.

11) Produits de nutrition thérapeutique contenant des compositions selon l'une quelconque des revendications 1 à 7, en association éventuelle avec un véhicule neutre convenant à l'administration orale ou entérale.

12) Compositions selon l'une quelconque des revendications 1 à 7 pour utilisation comme médicament.

13) Préparations pharmaceutiques contenant à titre de médicament des compositions selon l'une quelconque des revendications 1 à 7, en association éventuelle avec un véhicule neutre usuel convenant à l'administration orale ou entérale.

**Patentansprüche**

1. Kohlenhydrate, Vitamine, Mineralsalze, eine Lipidfraktion und eine Proteinfraktion enthaltende Zusammensetzungen, dadurch gekennzeichnet, daß sie Proteinfraktion drei Typen der folgenden Minipeptide umfaßt:
- Minipeptide des Lactoserums,
- Gesamt-Minipeptide des Caseins,
- Minipeptide des Caseins ohne Phosphopeptide.

2. Zusammensetzungen gemäß Anspruch 1, dadurch gekennzeichnet, daß das Gewicht der Proteinfraktion 1 bis 10 % des Gesamtgewichts ausmacht.

3. Zusammensetzungen gemäß Anspruch 1 oder 2, dadurch gekennzeichnet, daß der Gehalt an an Proteine gebundenem Phosphor zwischen 1,5 mg/g Protein und 6,6 mg/g Protein liegt.

4. Zusammensetzungen gemäß einem der Ansprüche 1 bis 3, dadurch gekennzeichnet, daß die drei Minipeptid-Typen im wesentlichen in äquivalenten Gewichtsanteilen vorliegen.

5. Zusammensetzungen gemäß Anspruch 4, dadurch gekennzeichnet, daß der Gehalt an an Proteine gebundenem Phosphor etwa 3 mg/g Protein beträgt.

6. Zusammensetzungen gemäß Anspruch 4, dadurch gekennzeichnet, daß die Verteilung an Aminosäuren der Proteinfraktion die nachstehend angegebene ist, wobei die folgenden Ziffern in Gramm Aminosäure je 100 g Aminosäuren ausgedrückt sind:

| Ile | 5,1 | Val | 5,9 |
|-----|-----|-----|-----|
| Leu | 10,1 | Arg | 3,3 |
| Lys | 8,5 | His | 2,5 |
| Met | 2,3 | Ala | 3,6 |
| Cys | 1,6 | Asp | 8,4 |
| Phe | 4,6 | Glu | 15,9 |
| Tyr | 4,9 | Gly | 1,8 |
| Thr | 4,4 | Pro | 8,4 |
| Trp | 1,8 | Ser | 4,7 |

7. Zusammensetzungen gemäß einem der Ansprüche 1 bis 6, dadurch gekennzeichnet, daß deren Lipidfraktion etwa die folgende Zusammensetzung besitzt:
- Triglyceride mit mittlerer Kette 56,4 %
- Önotheraöl 25,6 %
- Sojaöl 18 %

8. Verwendung der Zusammensetzungen gemäß einem der Ansprüche 1 bis 7 als Nahrungsmittel oder Nahrungsmittelzusatz entsprechend den spezifischen Ernährungsbedürfnissen.

9. Nahrungsmittel oder Nahrungsmittelzusatz enthaltend sie Zusammensetzungen gemäß einem der Ansprüche 1 bis 7, gegebenenfalls in Assoziation mit einem neutralen Träger, der der oralen oder enteralen Verabreichung zuträglich ist.

10. Zusammensetzungen gemäß einem der Ansprüche 1 bis 7 für die Verwendung als therapeutische Ernährungsprodukte.

11. Therapeutische Ernährungsprodukte, enthaltend die Zusammensetzungen gemäß einem der Ansprüche 1 bis 7, gegebenenfalls in Assoziation mit einem neutralen Träger, der für die orale oder enterale Verabreichung zuträglich ist.

12. Zusammensetzungen gemäß einem der Ansprüche 1 bis 7 für die Verwendung als Arzneimittel.

13. Pharmazeutische Zusammensetzungen, enthaltend als Arzneimittel Zusammensetzungen gemäß einem der Ansprüche 1 bis 7, gegebenenfalls in Assoziation mit einem üblichen, neutralen Träger, der für die orale oder enterale Verabreichung zuträglich ist.

**Claims**

1) Compositions containing glucides, vitamins, mineral salts, a lipid fraction and a protein fraction, characterized in that the protein fraction contains the following 3 types of minipeptides:
- lactoserum minipeptides,
- total casein minipeptides,
- casein minipeptides without phosphopeptides.

2) Compositions according to claim 1, characterized in that the weight of the protein fraction represents 1 % to 10 % of the total weight.

3) Compositions according to claim 1 or 2, characterized in that the level of phosphorus bound to proteins is between 1.5 mg per g of protein and 6.6 mg per g of protein.

4) Compositions according to any one of the claims 1 to 3, characterized in that the 3 types of minipeptides are present in approximately equivalent quantities by weight.

5) Compositions according to claim 4, characterized in that the level of phosphorus bound to proteins is approximately equal to 3 mg/g of protein.

6) Compositions according to claim 4, characterized in that the distribution in amino acids of the protein fraction is that given below, the following figures being expressed in grams of amino acid per 100 g of amino acids:

| Ile | 5.1 | Val | 5.9 |
|-----|-----|-----|-----|
| Leu | 10.1 | Arg | 3.3 |
| Lys | 8.5 | His | 2.5 |
| Met | 2.3 | Ala | 3.6 |
| Cys | 1.6 | Asp | 8.4 |
| Phe | 4.6 | Glu | 15.9 |
| Tyr | 4.9 | Gly | 1.8 |
| Thr | 4.4 | Pro | 8.4 |
| Trp | 1.8 | Ser | 4.7 |

7) Compositions according to any one of the claims 1 to 6, characterized in that their lipid fraction has approximately the following composotion:
- medium chain triglycerides 56.4 %
- Oenothera oil 25.6 %
- soya oil 18 %

8) Use of the compositions according to any one of the claims 5 to 7, as food or food supplement answering specific nutritional needs.

9) Food or food supplement containing compositions according to any one of the claims 1 to 7 possibly combined with a neutral vehicle suitable for oral or enteral administration.

10) Compositions according to any one of the claims 1 to 7, for use as therapeutic nutritional products.

11) Therapeutic nutritional products containing compositions according to any one of the claims 1 to 7, possibly combined with a neutral vehicle suitable for oral or enteral administration.

12) Compositions according to any one of the claims 1 to 7, for use as medicaments.

13) Compositions according to any one of the claims 1 to 7, possibly combined with a usual neutral vehicle suitable for oral or enteral administration.